# EUROPEAN PATENT SPECIFICATION

(11) **EP 4 240 220 B1**
(45) Date of publication and mention of the grant of the patent: **09.09.2026**
(21) Application number: 22745141.6
(22) Date of filing: 21.01.2022
(51) Int. Cl.: A61B 1/00, G02B 23/24, A61B 1/005, A61B 17/00, A61B 1/008, A61B 17/29

(54) **A STEERABLE ARM FOR USE IN ENDOSCOPIC SURGICAL PROCEDURES**
LENKBARER ARM ZUR VERWENDUNG BEI ENDOSKOPISCHEN CHIRURGISCHEN EINGRIFFEN
BRAS ORIENTABLE DESTINÉ À ÊTRE UTILISÉ DANS DES PROCÉDURES CHIRURGICALES ENDOSCOPIQUES

(30) Priority: 26.01.2021 US 202163141613 P
(43) Date of publication of application: 13.09.2023
(60) Divisional application: 26164351.4 / 4 732 792; 26165721.7 / 4 736 791
(73) Proprietor: Versitech Limited, Hong Kong (HK)
(72) Inventor: KWOK, Ka Wai, Hong Kong (CN); HE, Zhuoliang, Hong Kong (CN); WANG, Xiaomei, Hong Kong (CN); HO, Justin Di-Lang, Hong Kong (CN); FANG, Ge, Hong Kong (CN); WANG, Kui, Hong Kong (CN)
(74) Representative: McDonough, Jonathan
(86) International application number: PCT/CN2022/073108
(87) International publication number: WO 2022/161266

(56) References cited:
- WO-A1-2013/136583
- WO-A1-94/10897
- WO-A2-2004/103430
- CN-A- 104 968 255
- CN-A- 108 670 321
- CN-A- 111 658 152
- CN-A- 111 772 556
- CN-A- 112 022 060
- CN-U- 210 902 909
- US-A1- 2007 255 104
- US-A1- 2008 051 631
- US-A1- 2011 118 755
- US-A1- 2014 379 000
- US-A1- 2016 345 947
- US-A1- 2017 086 652
- US-A1- 2020 038 630
- US-B1- 6 171 316

## Description

### TECHNICAL FIELD OF THE INVENTION

The invention relates to the field of endoscopic surgical instruments.

### BACKGROUND

Gastrointestinal (GI) cancers are the most common cancers worldwide. According to World Health Organization (WHO) cancer statistics, colorectal and gastric cancers are respectively the second and third most common cause of cancer related deaths globally, which amount to 862,000 and 783,000 respectively in 2018. While advanced GI cancers are difficult to cure, early GI cancers carry a significantly better prognosis. The 5-year survival for GI cancers at an early stage is more than 90% worldwide.

Conventional treatment for GI cancers is surgery, which involves resection and anastomosis, and is associated with significant morbidity and mortality rates.

The preferred methods of surgery of the gastrointestinal (GI) tract include directing surgical instruments through the instrument/biopsy channels of an endoscope. A flexible endoscope is inserted through the mouth into the stomach to reach the target location, or from the anus up into the large intestine to reach the target location, with the distal end of the endoscope steered by the surgeon using a control handle on the proximal end of the endoscope. The endoscope has at least one biopsy channel into which a long, flexible instrument with a surgical tool at the distal tip is inserted from the proximal end of the endoscope. **The** most commonplace flexible endoscopes are made by Olympus, and have biopsy channel diameters as large as 3.7 mm, but which may be as small as 2.8 mm and lower.

Medrobotics proposes a semi-robotic robotic endoscope with channels for manual surgical instruments (described in US10016187B2). **The** diameter of the surgical instrument is assembled from many discrete joints to allow articulation when pulled by a pair of pair of antagonistic wires (two wires on opposite sides pulling in opposite directions). **To** bend a joint in one direction, one wire must be pulled, while the other wire must be released or pushed. **The** discrete joints are complex to design and assemble, and necessitate a wide 4-mm thick diameter. Hence, a major disadvantage of this surgical instrument is that it does not fit into the biopsy channel in the commonplace flexible endoscopes.

Endomaster proposes a similar robotic surgical instrument, which is also assembled from discrete joint-based mechanisms (described in US20210186309A1) that suffer from the same disadvantages.

Lumendi proposes an endoscopic surgical instrument that comprises a flexible backbone fabricated from a single body of flexible material, e.g. a nickel titanium (nitinol) tube, with unconnected, discrete slots cut into and along the side of the tube (described in US20200305906A1). **The** straight tube can be bent to either direction by pulling wires affixed to the respective side of the tube. Again, this design requires complex manipulation in pulling on one wire while releasing the opposite wire. Furthermore, this surgical instrument is also too thick to be useable with the biopsy channels in commonplace endoscopes and must be used with their proprietary accessory system.

Endotheia proposes a surgical instrument that can be used with the commonplace endoscope, described in US10441371B2. This design comprises nested concentric nitinol tubes, which are each pre-curved. These tubes have an overall diameter that is capable of extending through the biopsy channel in the commonplace endoscope. To bend the surgical instrument towards a target location, each of the nested tubes is extended to a suitable extent to create the required curvature. There is no need of wires to bend the tubes. However, the extent to which each curved tubes should extend requires complex computation, which makes it rather necessary for software and robotic control, and manual override is implausible even when the situation might call for it. Furthermore, the lifting force of the thin tubes is too weak for some procedures. Also, the tubes cannot provide a sharp bend, and the endoscope has to be placed relatively far from the target site in order for the tubes to extend far enough to provide sufficient curvature. As a result, the camera on the tip of the endoscope may be too far from the distal end of the surgical instrument to provide good visualization of the operation; the lateral and vertical reach of the instrument may be limited if the target site is too near the tip of endoscope.

US 2014/379000A1 (Romo, Enrique et al) discloses an instrument that facilitates bending with large degrees of articulation while maintaining ease of manufacturing for medical and surgical applications.

US 2017/0086652A1 (Nakade, Sho et al) discloses an endoscope insertion portion which includes: a first bending portion disposed on a distal end side; a second bending portion connected to a proximal end of the first bending portion and provided with flexural rigidity higher than flexural rigidity of the first bending portion; a tubular member, a first tubular part of which on a distal end side disposed inside the first bending portion has a bending tendency in a predetermined direction and shape-memorized, and which is provided with a second tubular part on a proximal end side disposed inside the second bending portion; a first wire configured to move the first tubular part and independently bend only the first bending portion; and second wires configured to move the second tubular part and independently bend only the second bending portion.

US 6171316B2 (Kovac, Tim J. et al) discloses an endoscopic surgical instrument suitable for use with a needle-grasper having a rotational translation device for creating rotational motion at the distal end of the surgical instrument in a non 1:1 ratio to rotational energy imparted at the handle of the surgical instrument. The shaft of the surgical instrument has an articulated segment capable of remote adjustment to articulate the effector unit at the end of the surgical instrument. The effector unit may be a needle-gripper having an upwardly pointed V-shaped notch in a channeled upper jaw, a convex gripping surface on the lower jaw, such that grasping a curved suture needle between the upper and lower jaw serves to automatically orient the needle perpendicular to the needle-gripper with the curvature pointed down. Conically tapered edges on the upper jaw further facilitate this orientation. A reciprocal motion transmission occurs between the handle and the effector unit so that gripping the levers on the handle serves to operate a function in the effector unit. The device is suitable for manual operation by a surgeon performing endoscopic surgical manipulations directly and without the intervention of motors or the like.

US 2020/0038630A1 (Besselink, Petrus A.) discloses a device, a method of making a device and a method of inserting a device into a tubular path. The device includes a tubular sheath with one or more helical slots formed therein and a control element that fits within the sheath. In one form, the device is an endoluminal device that simultaneously improves flexibility and structural rigidity though variations in one or both of slot width along the length of the slot and slot pitch along the length of the tubular sheath. When the operator pulls at the proximal end of the control element while holding the outer sheath in place, the slots will tend to close in a preferential manner such that more precise control of device length and bending is enabled, while simultaneously providing improvements in structural rigidity during device insertion and navigation through a body lumen or related tubular member where tortuous paths may be encountered along the member path.

US 2016/0345947A1 (Salahieh, Amr et al) discloses steerable medical devices and methods of use. In some embodiments, the steerable medical devices include a steerable portion with a stiffness that varies along the length of the steerable portion.

WO 2004/103430A2 (USGI Medical Corp.) discloses systems, devices and methods for endoscopic procedures involving tissue manipulations beyond the capabilities of traditional endoscopic instruments. Embodiments of the systems include an elongated main body having a scope therethrough. Some embodiments of the systems include an elongated main body which is rigidizable and/or torque transmitting to improve manipulation through passageways in the body.

US2008/051631A1 (Dejima, Takumi et al) discloses a medical treatment endoscope including a sheath having a flexibility; at least one arm member having a bending part that projects out from a front end of the sheath and performs bending actions; an open/close mechanism which directs the arm member from a direction along a central axis of the sheath to a direction deviated from the central axis of the sheath, and from a direction deviated from the central axis of the sheath to a direction along the central axis of the sheath; and a viewing device and an illuminating member that are disposed to the front end side of the sheath.

US2011/118755A1 (Cooper, Thomas G. et al) discloses a tool having a wrist mechanism that provides pitch and yaw rotation in such a way that the tool has no singularity in roll, pitch, and yaw. In one embodiment, a minimally invasive surgical instrument comprises an elongate shaft having a working end, a proximal end, and a shaft axis between the working end and the proximal end; and an end effector. A wrist member has a flexible tube including an axis extending through an interior surrounded by a wall. The wall of the flexible tube includes a plurality of lumens oriented generally parallel to the axis of the flexible tube. The wrist member has a proximal portion connected to the working end of the elongate shaft and a distal portion connected to the end effector. A plurality of actuation cables have distal portions connected to the end effector and extend from the distal portion through the lumens of the wall of the wrist member toward the elongate shaft to proximal portions which are actuatable to bend the wrist member in pitch rotation and yaw rotation.

Therefore, it is desirable to propose a surgical instrument that provides dexterous tissue manipulation while being suitable for use with commonplace endoscopes, and which provides a possibility of better control to the surgeon.

### STATEMENT OF INVENTION

The present invention provides a steerable arm for fitting into the instrument channel of an endoscope for use in endoscopic surgical procedures as defined by the features of independent claim 1. Preferred embodiments are defined in the dependent claims 2-11.

Advantageously, the invention provides the possibility that releasing the pull allows the bias to manifest and restores the steerable arm to the shape in the rest state. This makes the second wire in the prior art steerable art redundant. This one-wire approach is easier than the two-wire approach of the prior art that requires additional coordination between pulling one wire and releasing the other, and saves space inside the steerable arm for other components or wires.

### BRIEF DESCRIPTION OF DRAWINGS

It will be convenient to further describe the present invention with respect to the accompanying drawings that illustrate possible arrangements of the invention, in which like integers refer to like parts. Other embodiments of the invention are possible, and consequently the particularity of the accompanying drawings is not to be understood as superseding the generality of the preceding description of the invention.
Figure 1 shows a prior art for comparative purpose;
Figure 2 shows a prior art for comparative purpose;
Figure 3 shows a device that includes an embodiment not according to the invention;
Figure 4 shows two of the device of Figure 3 in use with an endoscope;
Figure 5 is a close-up view which shows an embodiment not according to the invention, the view being a part of that shown in Figure 4;
Figure 5a shows the embodiment not according to the invention of Figure 5 fitted with forceps;
Figure 5b shows the embodiment not according to the invention of Figure 5 fitted electrosurgical knives;
Figure 6 is a photograph taken by the camera of an endoscope of a prototype corresponding to the embodiment not according to the invention of Figure 5;
Figure 7 is an illustration of a more specific embodiment that is found in the embodiment not according to the invention of Figure 5;
Figure 8 is a schematic illustration of the workings of the embodiment not according to the invention of Figure 7;
Figure 9 is a schematic illustration of the fabrication of the embodiment not according to the invention of Figure 7;
Figure 10 shows a part of the embodiment not according to the invention of Figure 7;
Figure 11 shows a part of the embodiment not according to the invention of Figure 7;
Figure 12 is an embodiment not according to the invention and which is an alternative to that of Figure 7;
Figure 13 shows alternate views of the embodiment not according to the invention of Figure 12;
Figure 14 is shows alternate views of the embodiment not according to the invention of Figure 12;
Figure 15 shows a variation of the embodiment not according to the invention of Figure 12;
Figure 16 shows an embodiment based on the embodiment of Figure 7;
Figure 17 is a schematic illustration of the fabrication of the embodiment of Figure 16;
Figure 18 is a technical drawing of the embodiment of Figure 16;
Figure 19 is a schematic illustration showing how the embodiment of Figure 16 operates;
Figure 20 illustrates the operation of another embodiment;
Figure 21 shows a variation to the embodiment of Figure 20;
Figure 21a shows another variation to the embodiment of Figure 20;
Figure 22 shows a variation to the embodiment of Figure 7;
Figure 23 is a schematic illustration of the fabrication of the embodiment of Figure 22;
Figure 24 shows how the embodiment of Figure 22 operates;
Figure 25 shows a possible feature in the embodiment of Figure 7;
Figure 26 shows variation to the feature Figure 25; and
Figure 27 shows a more general embodiment not according to the invention.

### DESCRIPTION OF EMBODIMENT(S)

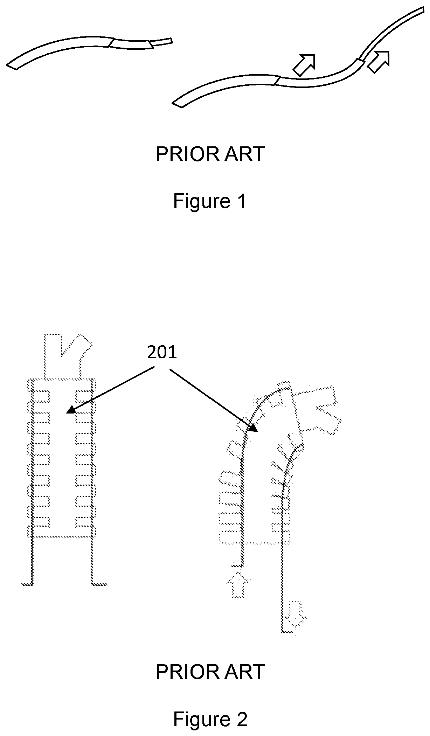

Figure 1 shows a prior art for comparative purpose. The prior art is a steerable arm that may be used in endoscopic surgery. This steerable arm comprises nested, curved segments that can extend telescopically. The end of the thinnest segment can extend furthest and is affixed with an end-effector, such as a pair of forceps or a diathermy knife. Each segment can be extended fully or partially to contribute the overall bend of the steerable arm in order to reach
a target area. Bending of the steerable arm is not achieved through pulling wires.

Figure 2 illustrates another steerable arm of the prior art. This steerable arm is generally an elongate member 201 that has a series of parts cut out from the lateral sides of the elongate member. Two wires inside the elongate member are affixed to opposite lateral sides at the distal end of the elongate member. To bend the elongate member to one side, one wire has to be pulled while the other wire has to be released, as an agonist-antagonistic wire pair.

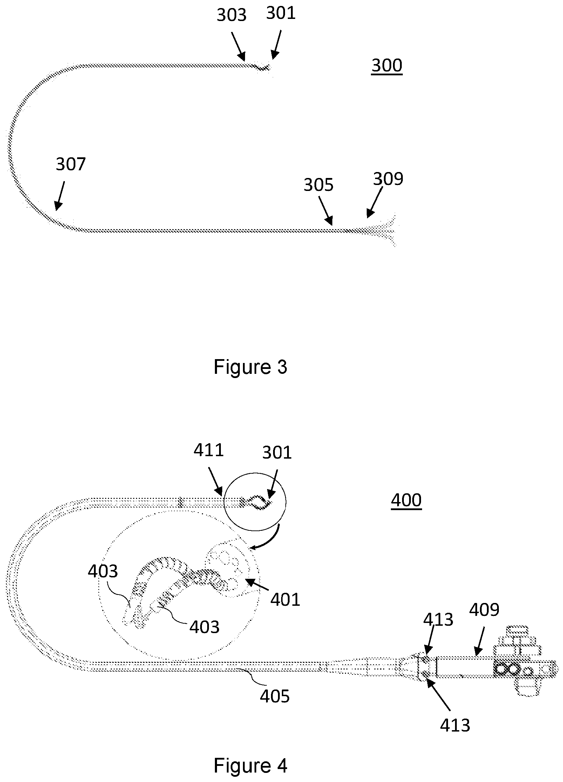

Figure 3 illustrates an embodiment not according to the invention, which is a flexible surgical instrument 300 for use with an endoscope 400.

The flexible surgical instrument 300 comprises a transmission tube 307, which makes up the bulk of the length of the flexible surgical instrument 300. The distal end 303 of the transmission tube 307 is installed with a steerable arm 301. In turn, the distal end of the steerable arm 301 is affixed with a surgical end-effector 403 (see insert in Figure 4) that determines the functionality of the flexible surgical instrument 300, such as forceps, diathermy knife, injection needle, suturing tool and so on.

Figure 4 shows an endoscope 400 inserted with two flexible surgical instruments 300. An endoscope 400 is an optical instrument that is capable of being extended into the gastrointestinal (GI) tract through the mouth or anus, and manoeuvred to reach a target location in the tract to provide a view. An endoscope 400 may comprise a video display connected to its proximal end, and a light source and a camera with a large field of view on the distal end 411. Image transmission from the camera to the video display may be
provided by an optical fibre system or a sensor chip system.

Endoscopes 400 for GI procedures are typically longer than 1 m. The outer diameter of an endoscope 400 that has two biopsy channels is usually larger than 1.2 cm. The core of the most common GI endoscopes 400 is provided with one or two channels that may have a diameter of 2.8 mm to 3.7 mm, typically called the biopsy channels 405 or instrument channels. A biopsy channel 405 has a channel entrance 413 at the proximal end of the endoscope 400 and a channel exit at the distal end 411 of the endoscope 400. The flexible surgical instrument 300 can be inserted into a biopsy channel 405 through the channel entrance. The endoscope 400 of Figure 4 has two biopsy channels 405, one for each of the two flexible surgical instruments 300.

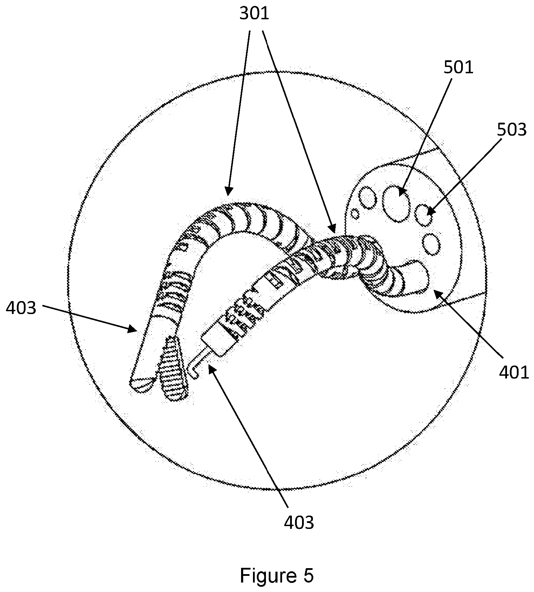

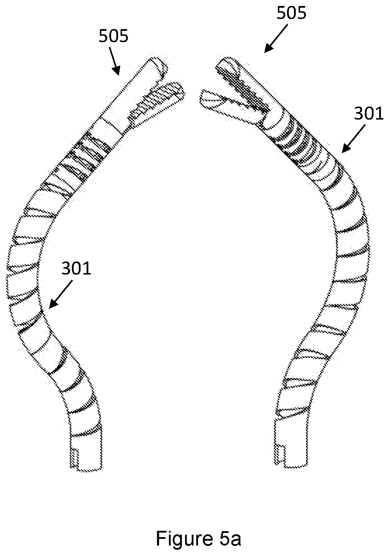

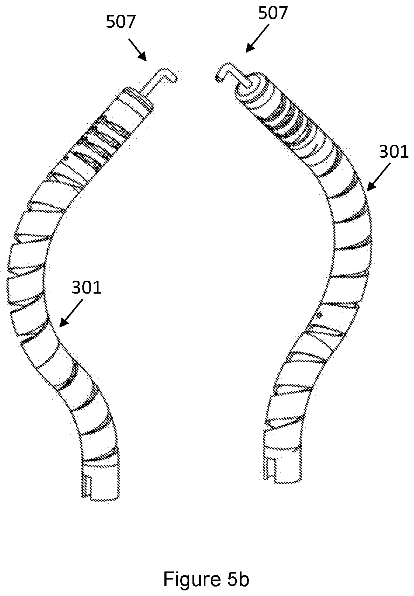

Figure 5 is a magnified view of the drawing insert in Figure 4 and shows an exemplary arrangement of the camera 501 and the source of light 503 on a cap 401 at the distal end 411 of the endoscope 400. The camera 501 provides a live view of the surgical site, steerable arms 301 and the end-effector 403, to guide the surgeon in manipulating the steerable arms 301.

Figure 5a shows a steerable arm 301 the distal end of which may be provided with forceps 505 as the end-effector. Figure 5b shows a steerable arm 301 the distal end of which may be provided with a diathermy knife 507 as the end-effector.

Other end effectors can be installed onto the distal tip of the steerable arm 301 to determine the final functionality of the arm, such as injection needle and suturing tools and so on.

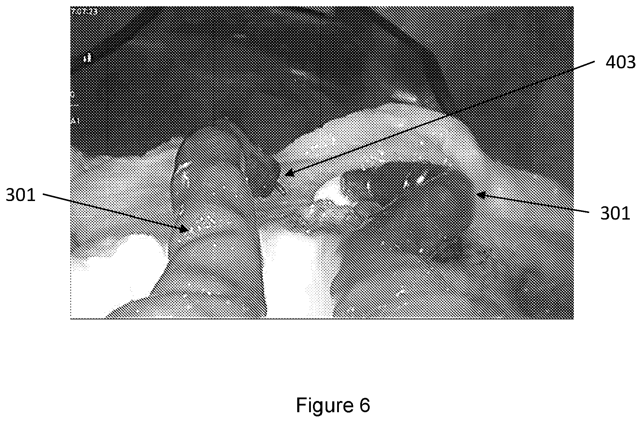

Figure 6 is a picture that may be taken by the camera 501, showing two steerable arms 301 each affixed with an end-effector 403 and performing a procedure on tissue. The two steerable arms 301 in Figure 6 may be fitted with an outer sheath, which reduces friction when the flexible surgical instrument 300 is inserted into the biopsy channel 405, and to provide electrical insulation.

In the preferred embodiment, the transmission tube 307 and the steerable arm 301 have an outer diameter of 2.7 mm or less, in order to fit into most biopsy channels 405 provided by commonplace GI endoscopes 400. The length of the transmission tube 307 may vary by design and depends on the length of the endoscope 400 that the flexible surgical instrument 300 is intended to be used with.

The steerable arm 301 can be moved or bent by pulling on wires that are threaded through at least one channel in the transmission tube 307. The distal ends of some of the wires are affixed to different parts inside the steerable arm 301. The distal ends of the remaining wires are connected to the end-effector 403.

The ends of the wires 309 protruding from the proximal end 305 of the flexible surgical instrument 300 are coupled to an adapter (not illustrated) located outside of the endoscope 400. The adapter comprises knobs, pulleys or levers (not illustrated) to which the ends of the wires are separately connected. Rotation or translation of each knob, pulley or lever causes a pull on the respective wire. Pulling on the proximal end of the wires move or bend the steerable arm 301, or actuate the end-effector 403. The adapter can be operated robotically via electronic components and software to control movements of the steerable arms 301 and the end-effectors 403.

In a surgery, the surgeon inserts the endoscope 400 into the patient's body and navigates the endoscope 400 to the desired location in the GI tract. The wires 309 that extend from the proximal end of the endoscope 400 can be pulled or released by the adapter.

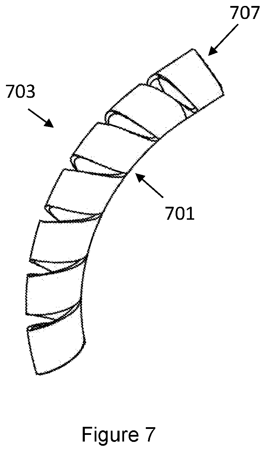

Figure 7 illustrates the main part of the steerable arm 301, which may be a metal tube fashioned into a tubular coil 707 of loops or a spiralling metal ribbon. The steerable arm 301 may be bent when at rest, and therefore has a convex side 703 and a concave side 701. On the concave side 701, the edges of the loops are closed up, and each edge abuts the corresponding edge of each adjacent loop, which prevents compression of the loops on the concave side 701. This provides a spine on the concave side 701 of the tubular coil 707. On the convex side 703, the edges of the loops are spaced apart, and this forms ribs that extend from the spine. The edges of the ribs on the concave side 701 are capable of moving closer or further apart from each other, to allow the spine to be flexed. The steerable arm 301 can be flexed to become straightened or, optionally, even bent to the opposite side. However, the metal is a resilient material and provides a bias in the steerable arm 301 to revert to the original shape when the flexing force is removed.

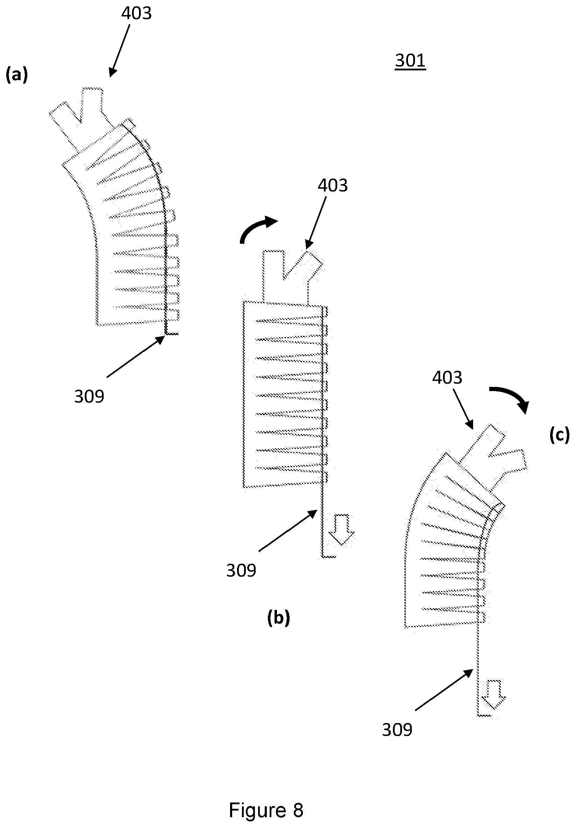

Figure 8 is a series of schematic illustrations of the articulation of the steerable arm 301. The drawings show the end of the wire 309 for controlling the steerable arm 301 extending through the core of the steerable arm 301 and connected to a rib at or near the distal end of the steerable arm 301. The wire 309 is illustrated as a solid line for clarity but the skilled reader would appreciate that the wire 309 is largely hidden from sight by being inside the steerable arm 301. The distal end of the wire 309 can be secured to the rib via a knot, crimp, or by other means of ensuring the wire 309 remains fixed to the rib. The steerable arm can be made of a spiral of loops or can be made from a tube which has sections cut along the side of the tube.

The left-most drawing shows the steerable arm 301 in the rest state, which is bent such that the spine is concave (Figure 8a). The ribs are on the convex side and spread apart to accommodate the bend. When the wire 309 is pulled, some of the ribs are brought closer to each other, and the spine is flexed and straightened (Figure 8b). On pulling the wire 309 further, the ribs are pulled even closer to each other such that the curvature of the steerable arm 301 reverses and now bends away from the original bending direction (Figure 8c). Releasing the pull allows the bias to manifest and restores the steerable arm 301 to the shape in the rest state. It is this bias that makes a second wire to pull the steerable arm back to its original rest state redundant.

Therefore, the steerable arm 301 can be moved from being bent in one direction to being bent in another direction within a plane. This allows the end-effector 403 on the steerable arm 301 to manipulate tissue. This one-wire approach is easier than the two-wire approach of the prior art that requires additional coordination between pulling one wire and releasing the other. Also, the presence of the second wire would take up space within the steerable arm 301.

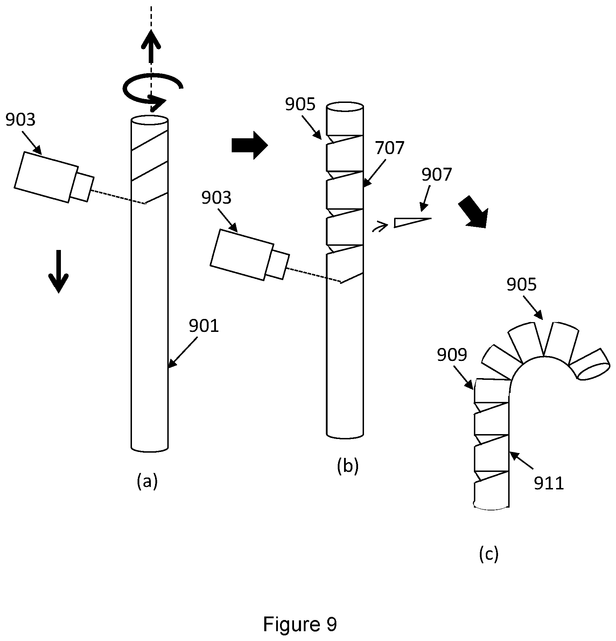

Figure 9 shows schematically a preferred way in which the steerable arm 301 is made. Firstly, a tube 901 made of a super-elastic material is provided, which has a diameter small enough to be channelled through the biopsy channel 405 of the endoscope 400 that the steerable arm 301 is expected to be used on. Typically, a diameter of 2.7 mm or less is preferred for universal application. The trailing transmission tube 307 may have a similarly small diameter that is not a concern for channelling through the biopsy channel. Super-elastic materials are those which have the ability to undergo large deformations and immediately return to its undeformed shape upon removal of the external load, such as nitinol (nickel titanium) and the following alloys: Cu-Zn, Cu-Al-Ni, Au-Cd, Au-Cu-Zn, and **In-TI.**

The tube 901 is cut spirally and along the length of the tube 901, as shown in Figure 9a, to fabricate the tube 901 into a tubular coil 707. The cut may be made by a precision machining e.g. laser cutting, computer numerical control (CNC) milling or other means. Figure 9 illustrates a laser source 903 used to cut the tube 901.

Other cuts are made into the side of the tube 901 to slice and remove a part 907 of each loop. This provides a gap 905 between every two adjacent loops on that side of the tube 901, which provides the ribs 909 (Figure 9b). The gaps 905 allow the ribs 909 to close in onto each other to accommodate a bend of the spine 911 toward the rib side. Also, the ribs 909 can be moved further apart to accommodate a bend of the spine 911 towards the spine side (Figure 9c). The other side of the tube 901 that forms the spine 911 is cut with minimal gaps 905 between the loops.

It should be noted that a spiral cut is made all around the circumference of the tube 901, including the side of the tube 901 that becomes the spine 911. The edges of the loops on the spine 911 are therefore also cut and separated. However, edge of each loop on the spine side abuts the edge of each adjacent loop. The abutment prevents compression of the spine 911 when the steerable arm 301 is flexed by pulling the wire 309.

Optionally, in other embodiments, the tube 901 can be cut only one side to provide gaps 905 that form the ribs 909, without a spiralling cut that is made all around the circumference, so that the side of the tube 901 that forms the spine 911 is left integral and not cut in any way at all.

Optionally, the steerable arm 301 is then held into the desired shape and heat-treated or plastically deformed to cause the material to memorise the shape.

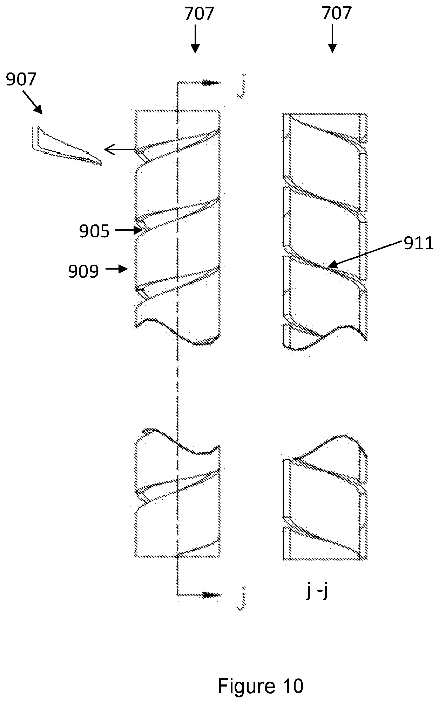

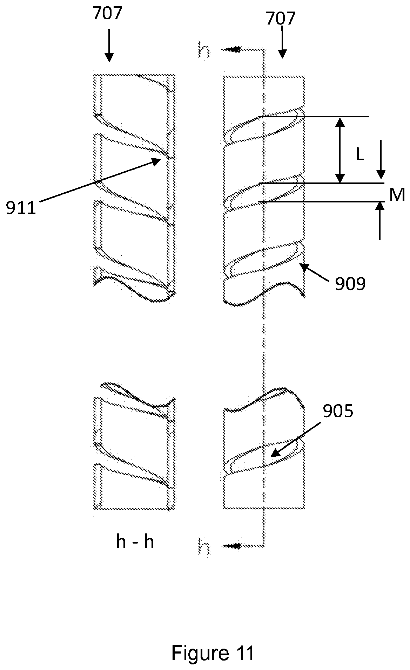

Figure 10 and Figure 11 are technical drawings of the ribs 909 and the spine 911, without the spine 911 shown bent. The left drawing in Figure 10 shows the exterior image of the steerable arm 301, while the right drawing of Figure 10 is the corresponding cross-sectional view from the direction marked h-h. The left of Figure 11 is the cross-sectional image in the direction j-j, while the right of Figure 11 shows the corresponding outward appearance of the steerable arm 301.

The spiral pattern can be varied to change the spiral pitch (L), spacing variation (M), number of turns, thickness of cut, shape of cut, slot location and size. These parameters can be adjusted to change the profile of the bending, range of motion, and stiffness of the spine 911, and may be selected according to surgical application requirements. Accordingly, the extent and shape of the pre-curvature can be varied to meet different surgical requirements such as a particular angle-of-attack of the instrument end-effector 403.

The stiffness, flexibility and resilience of the steerable arm can be adjusted by varying the structure of the gaps and the loops. For example, if the loops are thicker, the steerable arm has less flexibility and is stiffer. Alternatively, if the loops are narrow, the steerable arm has more flexibility. The size of the gaps between the loops, the width of each gap, the tilt of the gaps (spiral pitch), the closeness of the gaps, all may affect the flexibility and the resilience. Generally, the larger the gaps and the greater the density of the loops, the more deformable or flexible the tube. Even in embodiments where the steerable arm is not a coil of loops, but merely a tube with gaps that are sliced into the tube along the side of the tube, the same variations in the gaps can produce similar variation in the flexibility of the tube. Therefore, it is possible to produce a steerable arm that is more flexible at the distal end and less flexible at the proximal end, merely by cutting the distal end more finely to have either more fine loops or more cut-out portions, and the proximal end to have less finely cut loops or less cut-out portions. Advantageously, this prevents pulling of a wire attached to a more distal part of the steerable arm to cause unintentional deformation of a more proximal part; this allows the distal end not to lose control sensitivity over the proximal end, which makes the steerable arm more dextrous.

The steerable arm 301 is fabricated from a single body of material, e.g. metal, that provides a continuum structure. A continuum structure refers to something that is integral and bends in a continuous way by actually deforming the material, rather than by movement between separate but connected joints. In other words, a continuum structure is typically made of a single piece of material. Furthermore, a continuum structure has no sharp folds or folding bends that may create high stress concentrations, and a continuum structure may only have gradually curving or straight sides. In this way, the molecular or elemental structure of the material may provide the strength, stiffness and resilience and flexibility to the continuum structure.

The continuum structure avoids having to assemble separate parts into a steerable arm 301, and overcomes the challenge found in prior art wherein steerable instruments require labour-intensive assembly of complex individual parts or interconnecting joints, and which the parts' complexity may have a resultant size too large for use with the biopsy channels 405 of some endoscopes 400. Furthermore, this also reduces manufacturing time, complexity, and costs. In contrast to the embodiment, the prior art steerable arm comprised of individual parts cannot be made to bend in the rest state, as separate parts cannot be heated to retain a memory of a position. Furthermore, the material used in a continuum structure can give sufficient strength and stiffness to the steerable arm 301 to retract/lift tissue in a surgical setting, while an assembled structure made up of separate parts cannot exploit the strength of the material to do so.

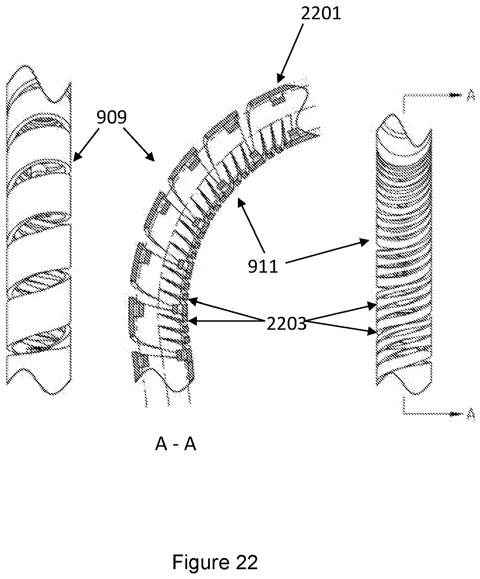

Preferably, eyelets 2201 (see Figure 22) for guiding the wire 309 are provided on the internal surface of the ribs. The eyelets allow axial translation of the wire 309 to facilitate actuation of the spine 911, and also constrain the wire 309 against the internal side of the steerable arm 301. The eyelets may be provided as additional parts fabricated separately and assembled together with the steerable arm.

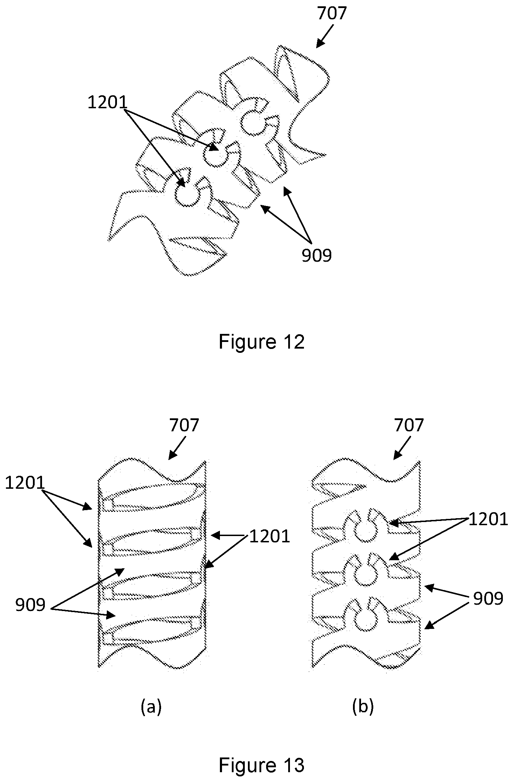

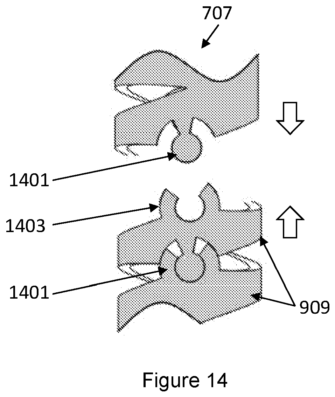

Figure 12 shows a second embodiment not according to the invention in which coupling joints 1201 are provided between every adjacent rib 909 to prevent the ribs 909 from widening or sliding radially or prevent twisting about the steerable arm long axis. Figure 13 and Figure 14 correspond to Figure 12, except that the version in Figure 12 is shown biased to bend in the rest state.

The coupling joints 1201 can be seen in Figure 13a as provided on opposite sides of the steerable arm 301. The edges of the ribs 909 are spaced apart on the convex side of the bend, as well as on the concave side. Thus, unlike the afore-mentioned embodiments, the spine is not defined by abutting ribs on the concave side. Instead, the spine is defined by the coupling joints 1201 which prevent the ribs 909 from being compressed, and the coupling joints 1201 are arranged in a column along the length of the steerable arm 301 such that the coupling joints 1201 provide the pivots about which the ribs 909 may rotate, and the spine may bend to either side. In other words, the ribs 909 and the spine are orthogonally arranged to each other with respect to the axis of the tube 901.

The coupling joints 1201 minimise bending or twisting outside of the joints' rotational plane, and prevents the loops that form the ribs 909 from loosening and enlarging radially. Generally, the coupling joints 1201 do not provide additional stiffness to the steerable arm 301 for returning to an original shape when the steerable arm 301 is flexed. The resilience and bias is still provided by the choice of material making up the steerable arm 301, and that the steerable arm 301 is a continuum structure.

A wire 309 (not illustrated in this drawing) extends within the steerable arm 301 and is affixed to an edge of one of the most distal ribs 909, where the edge is on the part of the rib 909 that is on the convex side. Pulling on the wire 309 can articulate the ribs 909 by rotating each rib 909 about the respective coupling joints 1201 and bending the steerable arm 301. Bending simply means changing the curvature of the arm. Bending could mean bending the arm from a straight configuration or straightening the arm from a bent configuration. The edges of the ribs 909 on the convex side of the spine close up while the edges of the ribs 909 on the concave side open up. Pulling further on the wire 309 reverses the bend such that the steerable arm 301 now bends in the opposite direction (not illustrated) . The steerable arm 301 naturally returns to the memorised shape when the pull on the wire 309 is released.

This embodiment not according to the invention is also made by cutting a single tube of nitinol. Pieces of the tube are cut away to create a coil of spaced out spiralling loops, and these loops forms the ribs 909 of the steerable arm 301. However, the cut out leaves behind the shape of a male coupling joint on one side of each rib 909, and the shape of a female coupling joint on the other side of the rib 909. In this case, 'side' does not refer to the lateral sides of the whole tube, but the sides of each rib 909 of the coil. The male coupling joint on the lower side of each rib 909 as shown in the drawing fits into the female coupling joint on the upper side of the adjacent rib. After being cut from the tube, the steerable arm 301 is held in the desired bend with all the coupling joints 1201 mated and heat treated to memorise the bend.

In some variations of this embodiment not according to the invention, both sides of the tube about the spine of coupling joints can be affixed with a wire each, one wire to bend the tube to either side. In these variations, the tube may or may not be pre-curved. Of course, having a pre-curved, resilient tube allows only one wire to flex the tube to be curved towards the opposite side and the original shape restored by the bias provided by the resilience.

Figure 14 shows the male coupling joint 1401 and female coupling joint 1403 separately, unmated, each provided on facing sides of adjacent ribs 909. The male coupling joint 1401 comprises a neck on which is a round head, and is capable of fitting into the female coupling joint 1403 which comprises a jaw defining a round gap. The rounded jaw is capable of rotating about the round head to allow the ribs 909 to articulate. Note that during fabrication of the continuum structure, the male coupling joint and female coupling joint are already mated, and that Figure 14 is for illustrative purposes only.

Advantageously, the embodiment of Figure 12 and which is not according to the invention combines the benefits of a continuum device (an integral device without any assembly of different parts, providing benefits such as fabrication simplicity, ease of miniaturization, selectable stiffness based on the tube material and/or structure, inherent antagonistic force due to compliance) with the benefits of a joint-based bending segment (such as mechanical constraints to minimize axial compression and out-of-plane bending) .

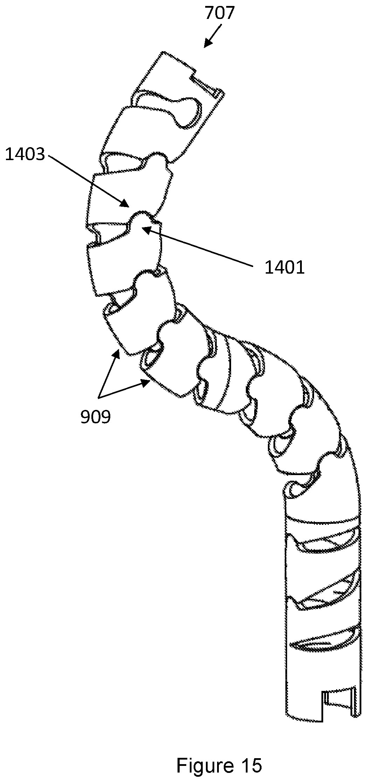

Figure 15 shows a variation of the coupling joints with a more simplified design. In this case, the male coupling joint 1401 is simply a small, rounded head without a neck, and the female coupling joint 1403 is a shallow cradle. There is no need for the female couple to claw onto the head of the make coupling joint as the male coupling joint does not have a neck.

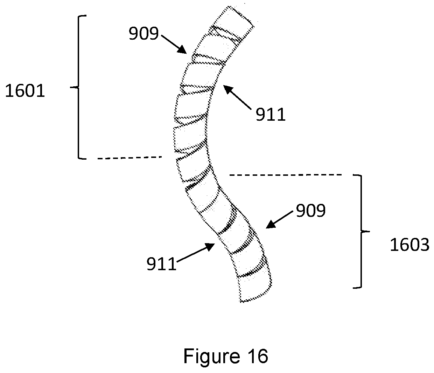

The embodiments described so far can be flexed to bend or be straightened within only one plane of movement and are not according to the invention. To provide multiple planes of movements, the embodiments can be thought of modularly, and be cut into different sections from the same nitinol tube as different parts of a larger steerable arm 301. Accordingly, Figure 16 shows a steerable arm 301 made of a single tube that has been cut such that there are two tubular coils 707 connected in series which are like that of Figure 7. The two tubular coils 707 shares the same axis but are angularly offset such that each part can be bent in a different plane.

Thus, the spine 911 of the top part 1601 of the tube (top as illustrated in the drawing) faces one direction while the spine 911 of the bottom part 1603 faces a different direction. The top part 1601 can be actuated by one wire 309 to bend and move in a first plane, while the bottom part 1603 can be actuated by another wire 309 to bend and move in a second plane. Moving the bottom part 1603 moves the top part 1601 too, as the top part 1601 extends from the bottom part 1603. In combination, this gives the surgeon greater freedom in directing the end-effector 403 on the steerable arm 301.

It should be noted that the meaning of being co-axial in the embodiments do not require the axis to be straight. The axis is curved and continuous along with the bent shapes of the steerable arms 301.

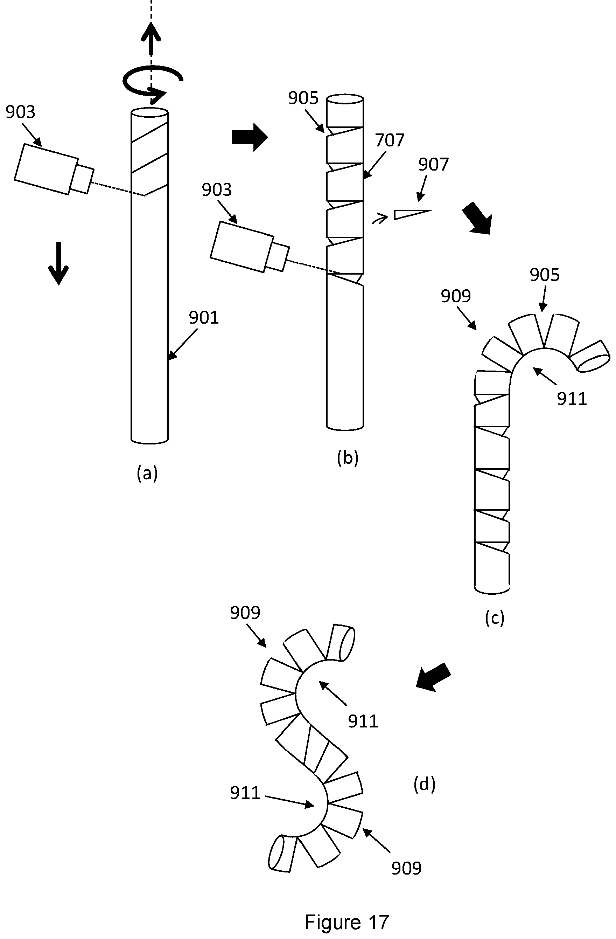

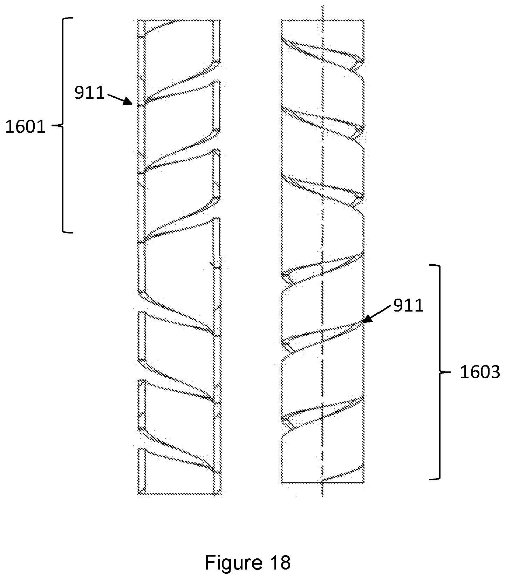

Figure 17 shows the method of fabrication not according to the claimed invention of the steerable arm 301 with multiple sections. Figure 17a illustrates how a spiralling cut is made along and around the whole tube 901 first, and produces a tubular coil 707 of loops. Subsequently, as shown in Figure 17b, a further cut is made into the side of the tubular coil 707 to remove a part of each loop in the top part. This provides the gaps 905 between the loops, forming the ribs 909. Such further cuts are also made to the bottom part of the tube, but to a different side of the tubular coil 707. As a result, the spine 911 of the bottom part and the spine 911 of the top part are on formed on different sides of the tube 901. Therefore, the top part is able to bend in one direction as shown in Figure 17c, while the bottom part is able to bend in a different direction as shown Figure 17d. Figure 18 is a technical drawing of the tube cut to provide a spine 911 in the top part 1601 which in different side to the spine 911 in the bottom part 1603. Finally, the top part 1601 is then held bent into the one desired shape and the bottom part 1603 is held bent into another desired shape, and heat treated (not illustrated) to memorise the overall shape.

Cutting the tube such that the spine of the top part and the spine of the bottom part bend in the exact opposite directions is just optional, as this means both the top part and the bottom part bend within the same plane albeit in opposite directions. Alternatively, that spine of the top part and the spine of the bottom part are angularly offset along the axis instead.

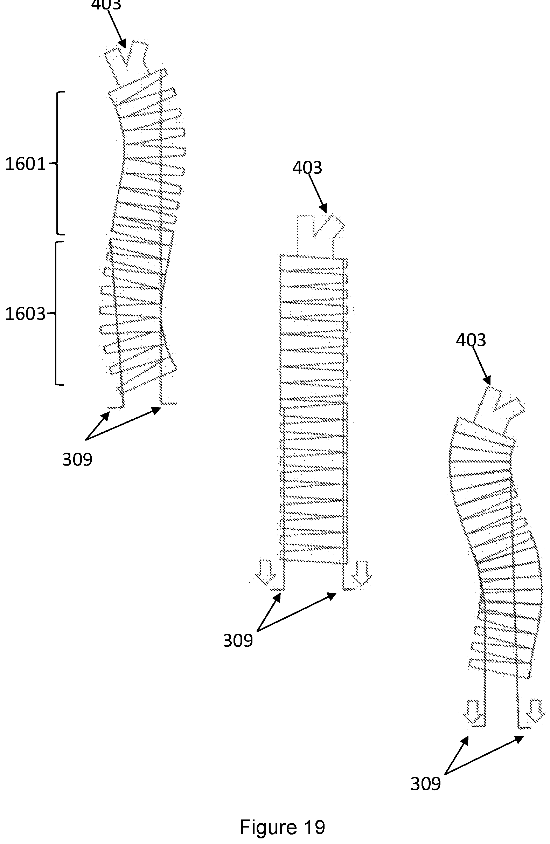

Figure 19 schematically shows how the embodiment of Figure 16 operates. In the drawing, the upper part 1601 of the steerable arm 301 is made to bend towards the left of the drawing when at rest. The lower part 1603 is made to bend to the right when at rest. As a result, the steerable arm 301 has a shape like an inverse letter S. The curvature of the upper part 1601 of the steerable arm 301 can be reduced by pulling on one wire 309 that extends inside the steerable arm 301, and which is fixed to one of the most distal ribs of the upper part 1601. When pulled further, the curvature can be reversed to be towards the rib-side.

Similarly, the lower part 1603 can be straightened when a wire 309 is pulled. If the wire 309 is pulled on further, the curvature can even be reversed to be towards the rib-side. In this case, the wire 309 extends inside the steerable arm 301 and is fixed to one of the ribs near the distal part of the lower part or, optionally, to the most proximal part of the upper part. This is because the distal part of the lower part 1603 ends where the proximal part of the upper part 1601 starts. Therefore, the most distal rib of the lower part 1603 is the rib just below the spine 911 of the upper part 1601.

When pulling fully on both wires 309, the inverse S shape of the steerable arm 301 is flipped, with all the ribs on both the upper part 1601 and lower part 1603 of the steerable arm 301 closed up.

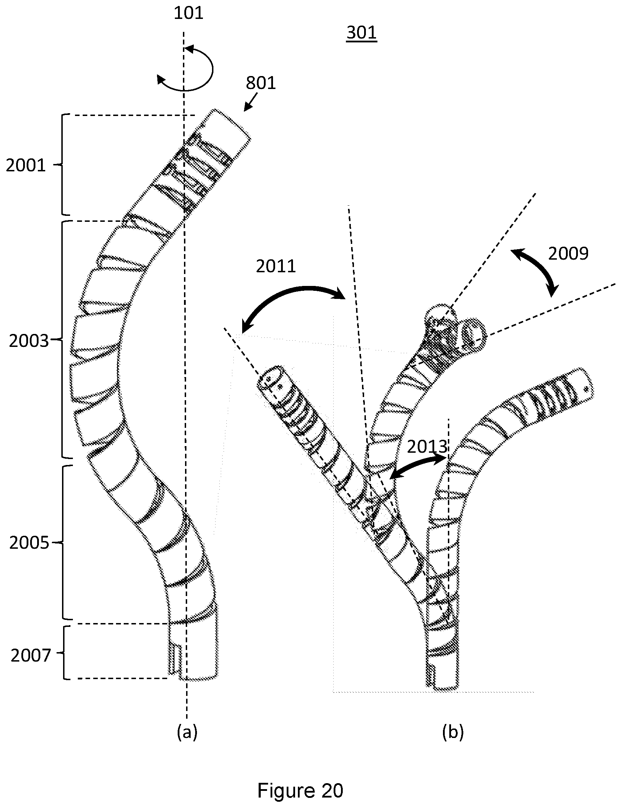

Figure 20 shows another embodiment which comprises different parts made up of the earlier embodiments. To fabricate the embodiment in Figure 20, different sections of the same metal tube is cut in a different way such that each section is able to bend in a different plane. A corresponding number of wires is provided inside the channel of the steerable arm 301, each wire 309 attached to the distal end of a respective section to control the bending of that section.

Figure 20a on the left of the drawing shows the steerable arm 301 in a rest state. Figure 20b on the right illustrates the different directions in which each part may be moved or bent by action of a respective wire 309.

There are four parts 2001, 2003, 20005, 2007 to the steerable arm 301 in Figure 20. The first, distal part 2001 of the steerable arm 301 is made like the embodiment of Figure 12. Right below this first part is a second part 2003 and third part 2005 which together correspond to the embodiment of Figure 16.

The first part 2001 and the second part 2003 are axially offset such that the first part 2001 is able to bend in a first plane 2009 while the second part 2003 is able to bend in a second plane 2011 that is at an angle to the first plane 2009. The second part 2003 and the third part 2005 are also axially offset such that the third part 2005 is able to bend in a third plane 2013 that is at an angle to the second plane 2011. Therefore, the three parts 2001, 2003, 2005 can be moved in different planes 2009, 2011, 2013, and provide three degrees of freedom of motion.

The fourth part 2007, which is below the third part 2005 as illustrated, is a coupler that is fitted to a corresponding coupler on the transmission tube 307 which can be affixed via various methods including welding, adhesive, or mechanically, etc. Preferably, the coupling allows the steerable arm 301 to rotate when the transmission tube is twisted at the proximal end of the endoscope, adding a further degree of movement.

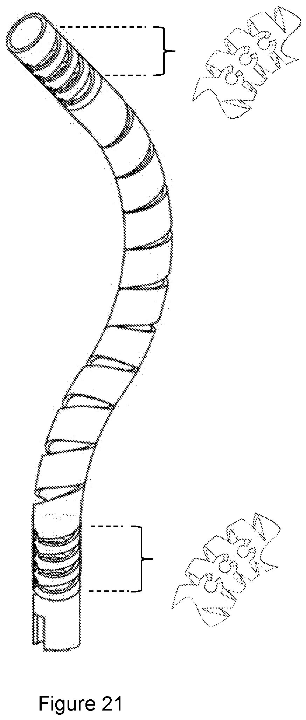

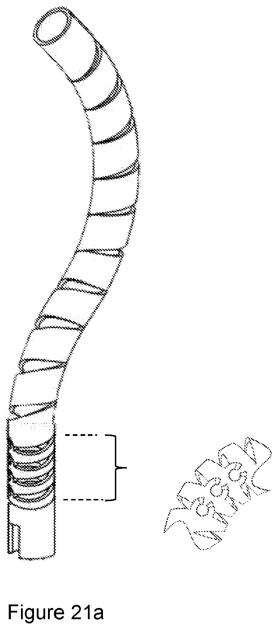

Figure 21 shows a variation of the embodiment of Figure 20. The main section of the steerable arm comprises the embodiment shown in Figure 16, the two ends of which are provide with the embodiment of Figure 12. The proximal end is provided with the coupler to connect the embodiment to the transmission tube. Thus, this steerable arm 301 has four different sections extending from the coupler, and at least two sections bend in a different plane.

In yet another embodiment shown in Figure 21a, the embodiment comprises coupler at the bottom, followed on top by a second section that is made according to the embodiment of Figure 12, which is in turn followed on top by the two-sectional embodiment of Figure 16. The end effector is wielded by the distal end of Figure 16 embodiment. In this embodiment, the two-sectional part is more flexible than the second section because the gaps in the two-section part are bigger. Similarly, the embodiment of Figure 15 has a more flexible distal section that is connected to a less flexible proximal section.

Figure 22 shows a further embodiment, in which the spine 911 in the embodiment of Figure 7 is provided with open slits 2203. The slits 2203 are not made around the entire circumference of the steerable arm 301, but are only cut into the spine 911 from the side of the steerable arm. Preferably, a slit 2203 is provided by removing a thin piece from the spine 911. The slits 2203 structurally reduces the resistance of the nitinol to allow the spine 911 to straighten up more easily when the wire 309 is pulling on the rib-side, resulting in improved dynamic performance. It should be noted that even in this case, the spine 911 resists compression significantly, despite the slits 2203. Optionally, the distal part of the embodiment can be given more cuts to make the distal part more flexible than the proximal part.

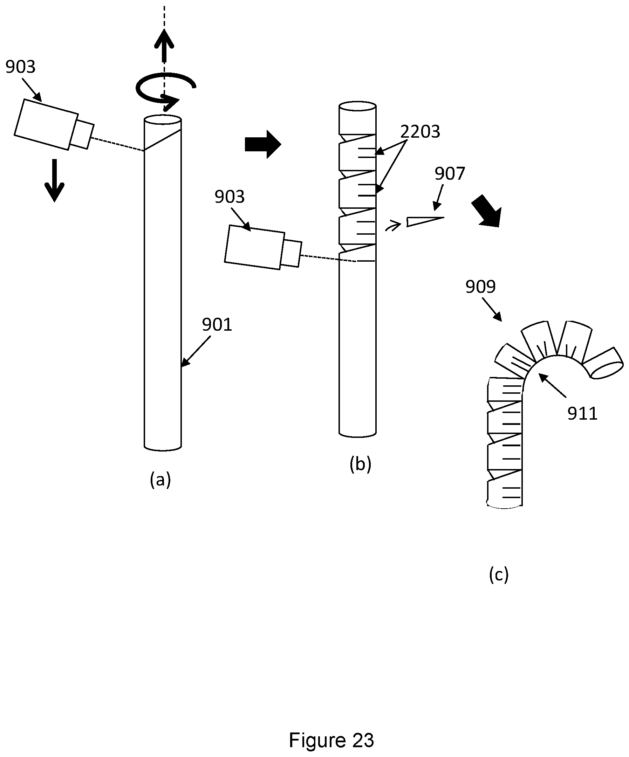

Figure 23 shows the process of cutting a tube 901 spirally with laser 903 to provide the ribs. The spiralling cut shown in Figure 23a is not made along the entire length of the tube 901 at once. If the spiral is cut at once along the entire length of the tube 901, the resultant tubular coil of loops will be too flimsy to allow slits to be cut into the spine. Instead, only a short section of the tube is spirally cut each time, followed immediately by slicing shallowing into side of the tube that is to become the spine. After that, the next part of the spiral cut is made by continuing from where the first spiral cut has stopped. Figure 23c shows how, after the tube has been satisfactorily cut, the resultant steerable arm 301 is bent into the desired shape and heat treated to memorizes the shape, where the spine is on the concave side and the ribs are on the convex side.

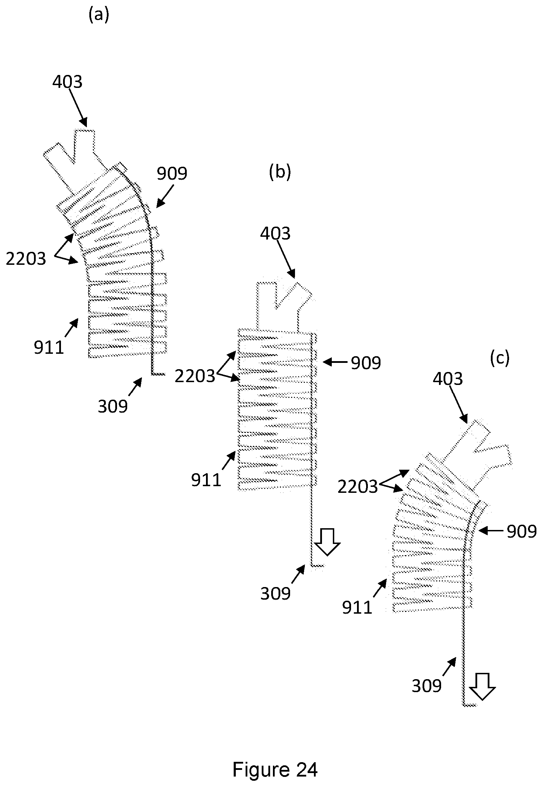

Figure 24 illustrates how the embodiment of Figure 22 operates. Figure 24(a) shows the rest state of the steerable arm 301 of Figure 22. Figure 24(b) shows the steerable arm 301 straightened and the slits open. Figure 24(c) shows the bend being reversed to flex to the opposite side with the slits on the spine more open.

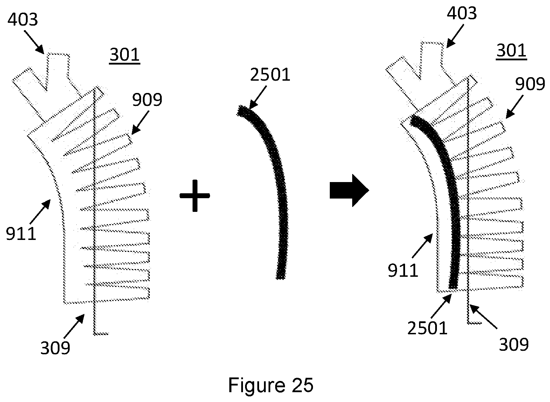

Figure 25 shows a variation of the embodiment of Figure 7, in which the stiffness of the spine 911 is reinforced by affixing the internal surface of the spine with a reinforcing element 2501, such as a stiff but bent piece of metal that conforms to the shape of the spine 911. The reinforcing element helps to restores the shape of the spine 911 quickly when the pull on the most distal rib 909 is released, which improves transmission response time and reduces mechanical hysteresis. The reinforcing element 2501 can be thought of as a spring. The reinforcing element is sufficiently flexible to allow straightening of the spine 911 when actuating the wire 309. The wire is not drawn as following the curve of the steerable arm in this embodiment, to show that is it not necessary in all cases that wire guides are provided to guide the translation of the wire.

Different sections of spine 911 in the embodiment of Figure 20 may be provided with such a reinforcing element each.

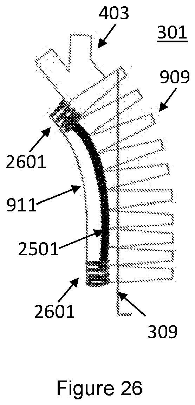

Figure 26 illustrates yet a further embodiment, wherein the strip of reinforcing element 2501 is not made of a very flexible material but both ends of the reinforcing element are supplied with springs 2601. The springs 2601 are secured to appropriate locations on the inside of the spine 911. The springs 2601 can be stretched when the steerable arm 301 is flexed. However, the springs 2601 reinforce the bias of the steerable arm 301 to restore memorised shape quickly when the pull on the most distal rib 909 is released. This improves transmission response time and reduces mechanical hysteresis, i.e. the effect of the pull of the wire 309 can be seen more readily in the steerable arm 301.

To further improve the responsiveness of the steerable arm 301, all the wires 309 connected to the steerable arms 301 are preferably pre-tensioned. That is, all the wires 309 are pulled taut in anticipation of use so that the sections of the steerable arm 301 are ready to be moved on further pulling of the wires 309. If the wires 309 were not pre-tensioned and hang loose, backlash would occur, resulting in a delay occurring before the steerable arm 301 responds to the wire pulling.

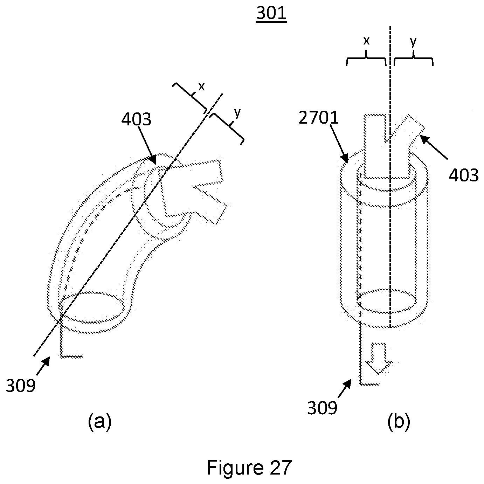

Figure 27 shows a more general embodiment which is not according to the invention, in which the steerable arm 301 comprises a hollow elongate member 2701 that is more resilient on one side of the axis, marked x, and more rigid on the other side of the axis, marked y. The elongate member is moulded, mould-cast or heat treated to be permanently bent towards the more rigid side when at rest. The material of the hollow elongate member is capable of remaining bent when at rest and yet is resilient enough to be straightened and even bent the other way by application of a suitable force. As with the previous embodiment, the curvature and bias in the bend provides the possibility of using one wire 309 to swing the steerable arm 301 across a plane. The hollow elongate member may be made of two different polymers that are co-extruded such that each polymer forms a side of the hollow elongate member. The convex side in this case can be provided with the more rigid material so that the steerable arm 301 does not shorten when the distal end of the steerable arm 301 is pulled upon by a wire 309. The concave side can be provided with the more expandable material that can stretch to accommodate the flexing of the steerable arm 301.

So far, the afore-described embodiments are described and illustrated to have a wire 309 secured to the side of a bent tube, the side being the furthest away from the spine. This provides better leverage when pulling the ribs close to
bend the spine. However, it is within the contemplation of this description that the wire is secured to the spine side of the bent tube.

Accordingly, the embodiments include a steerable arm 301 for use in an endoscope 400 to manipulate surgical tools as defined by the features of independent claim 1.

While there has been described in the foregoing description preferred embodiments of the present invention, it will be understood by those skilled in the technology concerned that many variations or modifications in details of design, construction or operation may be made.

For example, although loops have been mentioned as cut from a tube, it is possible that in some embodiments, that the ribs are just an extension from the spine that are curved, and are attached the spine on one end with the other end freely cantilevering.

For example, the flexible surgical instrument 300 can be adapted to be used with other kinds of devices that are similar to endoscopes 400, such as trans-
nasal endoscopes 400 or transurethral resectoscopes. Where these devices do not have an inner channel for inserting the flexible surgical instrument 300, an additional sheath can be made which maybe slipped over the device to create a channel for the flexible surgical instrument 300.

## Claims

1. A steerable arm (301) for fitting into the instrument channel of an endoscope (400) for use in endoscopic surgical procedures, comprising:
a tubular member having a proximal end and a distal end and at least two sections (1601, 1603, 2001, 2003, 2005, 2007);
the tubular member being a continuum structure made from a single piece of material and which is configured to bend in a continuous way by deforming the material;
the distal end suitable for being fitted with a surgical end effector (403);
the tubular member being made of a resilient material;
a respective number of wires (309) extending inside the tubular member from the proximal end;
the distal end of each section (1601, 1603, 2001, 2003, 2005, 2007) having a wire (309) affixed thereto, the distal end of the most distal section (1601, 1603, 2001, 2003, 2005, 2007) being the distal end of the tubular member;
each section (1601, 1603, 2001, 2003, 2005, 2007) capable of having a curvature lengthwise of the tubular member, such that curvature of each section (1601, 1603, 2001, 2003, 2005, 2007) is configured to change when the distal end of each section (1601, 1603, 2001, 2003, 2005, 2007) is pulled on by the respective wire (309), wherein
the resilience of the material is configured to provide the tubular member with a bias such that the curvature change of each section (1601, 1603, 2001, 2003, 2005, 2007) is reversed when the pull on the distal end of the each section (1601, 1603, 2001, 2003, 2005, 2007) is released; and
the change of curvature of each of the at least two sections (1601, 1603, 2001, 2003, 2005, 2007) lies in a different plane.

2. A steerable arm (301) for fitting into the instrument channel of an endoscope (400) for use in endoscopic surgical procedures, as claimed in claim 1, wherein
the tubular member has a first side and a second side along an axis of the tubular member;
the first side being more compressible than the second side;
wherein
the side of the tubular member that the wire (309) is attached to is the first side; anc
a pull on the wire (309) is configured to compress the first side to change the curvature of the tubular member.

3. A steerable arm (301) for fitting into the instrument channel of an endoscope (400) for use in endoscopic surgical procedures, as claimed in claim 2, further comprising gaps (905) in the first side configured to provide the compressibility of the first side; wherein the pull of the wire (309) is configured to change the curvature of the tubular member by bringing gaps (905) closer and bending the second side towards the first side.

4. A steerable arm (301) for fitting into the instrument channel of an endoscope (400) for use in endoscopic surgical procedures, as claimed in claim 3, further comprising
wire guides inside the tubular member for guiding the translation of the wire (309).

5. A steerable arm (301) for fitting into the instrument channel of an endoscope (400) for use in endoscopic surgical procedures, as claimed in any one of claim 2, claim 3 or claim 4, wherein
the tubular member is curved in a rest state, such that there is a concave side (701) and a convex side (703) to the tubular member; and
the convex side (703) of the tubular member being the first side.

6. A steerable arm (301) for fitting into the instrument channel of an endoscope (400) for use in endoscopic surgical procedures, as claimed in claim 5, wherein
the tubular member is a tubular coil (707) of spiralling loops providing the two sections;
the curvature of the tubular member being such that the edges of the loops on the first side are spaced apart to provide the gaps (905); and
the edge of each of the loops on the second side abutting the edge of each adjacent loop; wherein
the first side and the second side are on opposite sides of the tubular member.

7. A steerable arm (301) for fitting into the instrument channel of an endoscope (400) for use in endoscopic surgical procedures, as claimed in claim 6, wherein
the loops have different spiral pitches and/or spacing variations along different parts of the steerable arm (301), in order to provide the different parts with different flexibility.

8. A steerable arm (301) for fitting into the instrument channel of an endoscope (400) for use in endoscopic surgical procedures, as claimed in claim 6, wherein
the loops are produced by at least one spiral cut made to a tube (901).

9. A steerable arm (301) for fitting into the instrument channel of an endoscope (400) for use in endoscopic surgical procedures, as claimed in claim 6, further comprising
at least a slit in the edge of at least one of the loops on the second side.

10. A steerable arm (301) for fitting into the instrument channel of an endoscope (400) for use in endoscopic surgical procedures, as claimed in claim 1, wherein
the steerable arm (301) is placed at the distal end of a transmission tube (307);
the transmission tube (307) containing a channel (405) in which the wire (309) is threaded;
the proximal end of the wires (309) is connected to knobs and/or levers for pulling or the wire (309); such that
the curvature changes when the distal end of the tubular member is configured to be pulled on by the wire (309) at the proximal end of the transmission tube (307).

11. A steerable arm (301) for fitting into the instrument channel of an endoscope (400) for use in endoscopic surgical procedures, as claimed in claim 1, wherein the surgical end effector (403) is forceps (505), diathermy knife (507), injection needle, suturing tool.

## Patentansprüche

1. Lenkbarer Arm (301) zum Einsetzen in den Instrumentenkanal eines Endoskops (400) zur Verwendung bei endoskopischen chirurgischen Eingriffen, umfassend:
ein rohrförmiges Element, das ein proximales Ende und ein distales Ende und mindestens zwei Abschnitte (1601, 1603, 2001, 2003, 2005, 2007) aufweist;
wobei das rohrförmige Element eine Kontinuumsstruktur ist, die aus einem einzigen Materialstück hergestellt ist und die dazu konfiguriert ist, sich durch Verformen des Materials kontinuierlich zu biegen;
wobei das distale Ende dazu geeignet ist, mit einem chirurgischen Endeffektor (403) ausgestattet zu werden;
wobei das rohrförmige Element aus einem elastischen Material hergestellt ist;
eine jeweilige Anzahl von Drähten (309), die sich von dem proximalen Ende im Inneren des rohrförmigen Elements erstrecken;
wobei das distale Ende jedes Abschnitts (1601, 1603, 2001, 2003, 2005, 2007) einen daran befestigten Draht (309) aufweist, wobei das distale Ende des am weitesten distalen Abschnitts (1601, 1603, 2001, 2003, 2005, 2007) das distale Ende des rohrförmigen Elements ist;
wobei jeder Abschnitt (1601, 1603, 2001, 2003, 2005, 2007) in der Lage ist, eine Krümmung in Längsrichtung des rohrförmigen Elements aufzuweisen, sodass die Krümmung jedes Abschnitts (1601, 1603, 2001, 2003, 2005, 2007) dazu konfiguriert ist, sich zu ändern, wenn an dem distalen Ende jedes Abschnitts (1601, 1603, 2001, 2003, 2005, 2007) durch den jeweiligen Draht (309) gezogen wird, wobei
die Elastizität des Materials dazu konfiguriert ist, dem rohrförmigen Element eine Vorspannung bereitzustellen, sodass die Krümmungsänderung jedes Abschnitts (1601, 1603, 2001, 2003, 2005, 2007) umgekehrt wird, wenn der Zug an dem distalen Ende jedes Abschnitts (1601, 1603, 2001, 2003, 2005, 2007) gelöst wird; und
die Krümmungsänderung jedes der mindestens zwei Abschnitte (1601, 1603, 2001, 2003, 2005, 2007) in einer anderen Ebene liegt.

2. Lenkbarer Arm (301) zum Einsetzen in den Instrumentenkanal eines Endoskops (400) zur Verwendung bei endoskopischen chirurgischen Eingriffen nach Anspruch 1, wobei
das rohrförmige Element eine erste Seite und eine zweite Seite entlang einer Achse des rohrförmigen Elements aufweist;
die erste Seite komprimierbarer als die zweite Seite ist;
wobei
die Seite des rohrförmigen Elements, an der der Draht (309) befestigt ist, die erste Seite ist; und
ein Zug an dem Draht (309) dazu konfiguriert ist, die erste Seite zu komprimieren, um die Krümmung des rohrförmigen Elements zu ändern.

3. Lenkbarer Arm (301) zum Einsetzen in den Instrumentenkanal eines Endoskops (400) zur Verwendung bei endoskopischen chirurgischen Eingriffen nach Anspruch 2, ferner umfassend
Spalten (905) in der ersten Seite, die dazu konfiguriert sind, die Komprimierbarkeit der ersten Seite bereitzustellen; wobei
der Zug des Drahts (309) dazu konfiguriert ist, die Krümmung des rohrförmigen Elements zu ändern, indem Spalten (905) angenähert werden und die zweite Seite zu der ersten Seite hin gebogen wird.

4. Lenkbarer Arm (301) zum Einsetzen in den Instrumentenkanal eines Endoskops (400) zur Verwendung bei endoskopischen chirurgischen Eingriffen nach Anspruch 3, ferner umfassend
Drahtführungen im Inneren des rohrförmigen Elements zum Führen der Verschiebung des Drahts (309).

5. Lenkbarer Arm (301) zum Einsetzen in den Instrumentenkanal eines Endoskops (400) zur Verwendung bei endoskopischen chirurgischen Eingriffen nach einem von Anspruch 2, Anspruch 3 oder Anspruch 4, wobei
das rohrförmige Element in einem Ruhezustand gekrümmt ist, sodass es eine konkave Seite (701) und eine konvexe Seite (703) des rohrförmigen Elements gibt; und
die konvexe Seite (703) des rohrförmigen Elements die erste Seite ist.

6. Lenkbarer Arm (301) zum Einsetzen in den Instrumentenkanal eines Endoskops (400) zur Verwendung bei endoskopischen chirurgischen Eingriffen nach Anspruch 5, wobei
das rohrförmige Element eine rohrförmige Spule (707) aus spiraligen Schlaufen ist, die die zwei Abschnitte bereitstellen;
die Krümmung des rohrförmigen Elements derart ist, dass die Kanten der Schlaufen auf der ersten Seite beabstandet sind, um die Spalten (905) bereitzustellen; und
die Kante jeder der Schlaufen auf der zweiten Seite an die Kante jeder benachbarten Schlaufe anstößt; wobei
die erste Seite und die zweite Seite auf gegenüberliegenden Seiten des rohrförmigen Elements liegen.

7. Lenkbarer Arm (301) zum Einsetzen in den Instrumentenkanal eines Endoskops (400) zur Verwendung bei endoskopischen chirurgischen Eingriffen nach Anspruch 6, wobei
die Schlaufen unterschiedliche Spiralsteigungen und/oder Abstandsvariationen entlang verschiedener Teile des lenkbaren Arms (301) aufweisen, um den verschiedenen Teilen unterschiedliche Flexibilität bereitzustellen.

8. Lenkbarer Arm (301) zum Einsetzen in den Instrumentenkanal eines Endoskops (400) zur Verwendung bei endoskopischen chirurgischen Eingriffen nach Anspruch 6, wobei
die Schlaufen durch mindestens einen spiralförmigen Schnitt, der an einem Rohr (901) vorgenommen wird, hergestellt werden.

9. Lenkbarer Arm (301) zum Einsetzen in den Instrumentenkanal eines Endoskops (400) zur Verwendung bei endoskopischen chirurgischen Eingriffen nach Anspruch 6, ferner umfassend
mindestens einen Schlitz in der Kante mindestens einer der Schlaufen auf der zweiten Seite.

10. Lenkbarer Arm (301) zum Einsetzen in den Instrumentenkanal eines Endoskops (400) zur Verwendung bei endoskopischen chirurgischen Eingriffen nach Anspruch 1, wobei
der lenkbare Arm (301) an dem distalen Ende eines Übertragungsrohrs (307) platziert ist;
das Übertragungsrohr (307) einen Kanal (405) enthält, in den der Draht (309) eingefädelt ist;
das proximale Ende der Drähte (309) mit Knöpfen und/oder Hebeln zum Ziehen an dem Draht (309) verbunden ist; sodass
die Krümmung sich ändert, wenn das distale Ende des rohrförmigen Elements so konfiguriert ist, dass an ihm durch den Draht (309) an dem proximalen Ende des Übertragungsrohrs (307) gezogen wird.

11. Lenkbarer Arm (301) zum Einsetzen in den Instrumentenkanal eines Endoskops (400) zur Verwendung bei endoskopischen chirurgischen Eingriffen nach Anspruch 1, wobei der chirurgische Endeffektor (403) eine Zange (505), ein Diathermiemesser (507), eine Injektionsnadel, ein Nähwerkzeug ist.

## Revendications

1. Bras orientable (301) destiné à s'adapter dans le canal d'instrument d'un endoscope (400) destiné à être utilisé dans des procédures chirurgicales endoscopiques, comprenant :
un élément tubulaire possédant une extrémité proximale et une extrémité distale et au moins deux sections (1601, 1603, 2001, 2003, 2005, 2007) ;
l'élément tubulaire étant une structure continue fabriquée à partir d'une seule pièce de matériau et qui est conçue pour se plier de façon continue en déformant le matériau ;
l'extrémité distale adaptée à être équipée d'un effecteur terminal chirurgical (403) ;
l'élément tubulaire étant constitué d'un matériau élastique ;
un nombre respectif de fils (309) s'étendant à l'intérieur de l'élément tubulaire à partir de l'extrémité proximale ;
l'extrémité distale de chaque section (1601, 1603, 2001, 2003, 2005, 2007) comportant un fil (309) fixé à celle-ci, l'extrémité distale de la section la plus distale (1601, 1603, 2001, 2003, 2005, 2007) étant l'extrémité distale de l'élément tubulaire ;
chaque section (1601, 1603, 2001, 2003, 2005, 2007) étant capable de présenter une courbure dans le sens de la longueur de l'élément tubulaire, de sorte que la courbure de chaque section (1601, 1603, 2001, 2003, 2005, 2007) soit conçue pour changer lorsque l'extrémité distale de chaque section (1601, 1603, 2001, 2003, 2005, 2007) est tirée par le fil respectif (309),
ladite résilience du matériau étant conçue pour fournir à l'élément tubulaire un biais de sorte que le changement de courbure de chaque section (1601, 1603, 2001, 2003, 2005, 2007) soit inversé lorsque la traction sur l'extrémité distale de chaque section (1601, 1603, 2001, 2003, 2005, 2007) est relâchée ; et
ledit changement de courbure de chacune desdites au moins deux sections (1601, 1603, 2001, 2003, 2005, 2007) se situant dans un plan différent.

2. Bras orientable (301) destiné à s'adapter dans le canal d'instrument d'un endoscope (400) destiné à être utilisé dans des procédures chirurgicales endoscopiques, selon la revendication 1,
ledit élément tubulaire présentant un premier côté et un second côté le long d'un axe de l'élément tubulaire ;
ledit premier côté étant plus compressible que le second côté ;
ledit côté de l'élément tubulaire auquel le fil (309) est fixé étant le premier côté ; et
une traction sur le fil (309) étant conçue pour comprimer le premier côté de manière à modifier la courbure de l'élément tubulaire.

3. Bras orientable (301) destiné à s'adapter dans le canal d'instrument d'un endoscope (400) destiné à être utilisé dans des procédures chirurgicales endoscopiques, selon la revendication 2, comprenant en outre des espaces (905) dans le premier côté conçus pour assurer la compressibilité du premier côté ;
ladite traction du fil (309) étant conçue pour modifier la courbure de l'élément tubulaire en rapprochant les espaces (905) et en pliant le second côté vers le premier côté.

4. Bras orientable (301) destiné à s'adapter dans le canal d'instrument d'un endoscope (400) destiné à être utilisé dans des procédures chirurgicales endoscopiques, selon la revendication 3, comprenant en outre des guides de fil à l'intérieur de l'élément tubulaire pour guider la translation du fil (309).

5. Bras orientable (301) destiné à s'adapter dans le canal d'instrument d'un endoscope (400) destiné à être utilisé dans des procédures chirurgicales endoscopiques, selon l'une quelconque de la revendication 2, de la revendication 3 ou de la revendication 4,
ledit élément tubulaire étant incurvé dans un état de repos, de sorte qu'il y ait un côté concave (701) et un côté convexe (703) par rapport à l'élément tubulaire ; et
ledit côté convexe (703) de l'élément tubulaire étant le premier côté.

6. Bras orientable (301) destiné à s'adapter dans le canal d'instrument d'un endoscope (400) destiné à être utilisé dans des procédures chirurgicales endoscopiques, selon la revendication 5,
ledit élément tubulaire étant un serpentin tubulaire (707) de boucles en spirale fournissant les deux sections ;
ladite courbure de l'élément tubulaire étant telle que les bords des boucles sur le premier côté sont espacés pour fournir les espaces (905) ; et
ledit bord de chacune des boucles du second côté venant buter contre le bord de chaque boucle adjacente ;
ledit premier côté et ledit second côté étant sur des côtés opposés de l'élément tubulaire.

7. Bras orientable (301) destiné à s'adapter dans le canal d'instrument d'un endoscope (400) destiné à être utilisé dans des procédures chirurgicales endoscopiques, selon la revendication 6,
lesdites boucles présentant des pas en spirale différents et/ou des variations d'espacement le long de différentes parties du bras orientable (301), afin de fournir aux différentes parties une flexibilité différente.

8. Bras orientable (301) destiné à s'adapter dans le canal d'instrument d'un endoscope (400) destiné à être utilisé dans des procédures chirurgicales endoscopiques, selon la revendication 6,
lesdites boucles étant produites par au moins une coupe en spirale réalisée sur un tube (901).

9. Bras orientable (301) destiné à s'adapter dans le canal d'instrument d'un endoscope (400) destiné à être utilisé dans des procédures chirurgicales endoscopiques, selon la revendication 6, comprenant en outre
au moins une fente dans le bord d'au moins l'une des boucles sur le second côté.

10. Bras orientable (301) destiné à s'adapter dans le canal d'instrument d'un endoscope (400) destiné à être utilisé dans des procédures chirurgicales endoscopiques, selon la revendication 1,
ledit bras orientable (301) étant placé au niveau de l'extrémité distale d'un tube de transmission (307) ;
ledit tube de transmission (307) contenant un canal (405) dans lequel le fil (309) est fileté ;
ladite extrémité proximale des fils (309) étant reliée à des boutons et/ou des leviers permettant de tirer sur le fil (309) ; de sorte que
ladite courbure change lorsque l'extrémité distale de l'élément tubulaire est conçue pour être tirée par le fil (309) au niveau de l'extrémité proximale du tube de transmission (307).

11. Bras orientable (301) destiné à s'adapter dans le canal d'instrument d'un endoscope (400) destiné à être utilisé dans des procédures chirurgicales endoscopiques, selon la revendication 1, ledit effecteur terminal chirurgical (403) étant une pince (505), un couteau de diathermie (507), une aiguille d'injection, un outil de suture.
